(19) Europäisches Patentamt
European Patent Office
Office européen des brevets

(11) EP 0 617 028 B1

(12) **FASCICULE DE BREVET EUROPEEN**

(45) Date de publication et mention de la délivrance du brevet:
**14.06.2000 Bulletin 2000/24**

(51) Int. Cl.$^7$: **C07D 295/22**, C07D 295/06, C07D 295/08

(21) Numéro de dépôt: **94870043.0**

(22) Date de dépôt: **09.03.1994**

(54) **Enantiomères de la 1-[(4-chlorophényl)phénylméthyl]-4-[(4-méthylphényl)sulfonyl] pipérazine**
Enantiomere von 1-[(4-Chlorophenyl)phenylmethyl]-4-[(4-Methylphenyl)sulfonyl]piperazin
Enantiomers of 1-[(4-chlorophenyl)phenylmethyl]-4-[(4-methylphenyl)sulfonyl] piperazine

(84) Etats contractants désignés:
**AT BE CH DE DK ES FR GB GR IE IT LI LU MC NL PT SE**
Etats d'extension désignés:
**SI**

(30) Priorité: **15.03.1993 GB 9305282**

(43) Date de publication de la demande:
**28.09.1994 Bulletin 1994/39**

(60) Demande divisionnaire:
**99109564.7 / 0 955 295**

(73) Titulaire: **UCB, S.A.**
**1070 Bruxelles (BE)**

(72) Inventeurs:
- **Cossement, Eric**
  **B-1050 Bruxelles (BE)**
- **Bodson, Guy**
  **B-6739 Bellefontaine (BE)**
- **Gobert, Jean**
  **B-1040 Bruxelles (BE)**

(74) Mandataire:
**Lechien, Monique et al**
**UCB, S.A.,**
**Allée de la Recherche 60**
**1070 Bruxelles (BE)**

(56) Documents cités:
**EP-A- 0 058 146**
**BE-A- 523 901**
**GB-A- 2 225 321**
**US-A- 3 406 174**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.16.7 (HRS)/3.6

## Description

La présente invention se rapporte à des composés nouveaux, les énantiomères lévogyre et dextrogyre, substantiellement optiquement purs, de la 1-[(4-chlorophényl)phénylméthyl]-4-[(4-méthylphényl)sulfonyl]pipérazine de formule

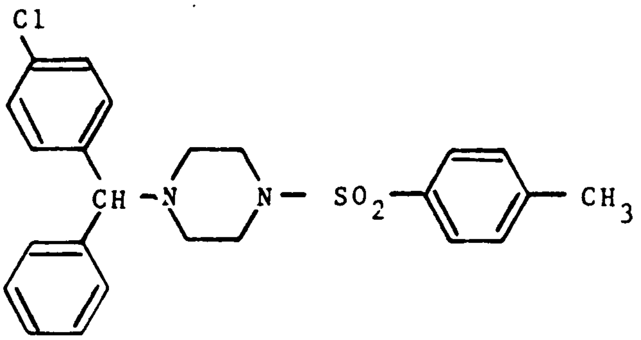

(I)

à un procédé de préparation de ces composés, de même qu'à leur utilisation pour la préparation des énantiomères lévogyre et dextrogyre substantiellement optiquement purs de la 1-[(4-chlorophényl)phénylméthyl]pipérazine. Ces derniers composés sont des produits intermédiaires précieux pour la préparation de composés thérapeutiquement actifs sous les formes lévogyre et dextrogyre substantiellement optiquement purs.

Ces composés thérapeutiquement actifs peuvent être utilisés dans le traitement de l'asthme, des allergies, de l'inflammation, de l'anxiété et comme agents sédatifs ou tranquillisants. Une propriété qui est fréquemment observée chez ces composés est leur activité antihistaminique périphérique et/ou centrale importante qui est à l'origine de leur utilisation comme médicaments.

Il est bien connu que les propriétés biologiques de nombreux composés, comme par exemple des médicaments, des hormones, des herbicides, des insecticides ou des édulcorants sont influencées par des facteurs stéréochimiques. L'importance des relations entre l'activité optique et les propriétés biologiques a été soulignée dès 1926 (A.R. CUSHNY, Biological Relations of Optically Isomeric Substances, Williams and Williams Co., Baltimore, 1926). Depuis lors, de nombreux exemples n'ont cessé d'affluer pour appuyer le principe généralement accepté maintenant qu'un composé racémique et ses énantiomères lévogyre et dextrogyre doivent être considérés comme des entités pharmacologiques entièrement distinctes. L'activité optique qui est un reflet de la structure asymétrique d'un composé organique, constitue l'un des facteurs importants qui modulent l'activité pharmacologique de ce composé et sa réponse biologique. En effet, selon que l'on utilise la forme lévogyre ou dextrogyre d'un composé à activité pharmacologique, des modifications profondes des propriétés du composé, telles que son transport, sa distribution dans l'organisme ou son élimination peuvent apparaître. Ces propriétés sont décisives pour la concentration du médicament dans l'organisme ou son temps d'exposition sur le site d'action. De plus, l'activité pharmacologique des deux isomères peut être considérablement différente. Par exemple, l'un des isomères optiquement actifs peut être nettement plus actif que l'autre et, à la limite, cet isomère pourrait posséder toute l'activité pharmacologique à lui seul, l'autre isomère étant totalement inactif et jouant alors le rôle de simple diluant. Il peut arriver aussi que les activités pharmacologiques des deux isomères soient différentes, ce qui fournit alors deux composés ayant des propriétés thérapeutiques distinctes. En outre, le métabolisme et la toxicité peuvent être fortement différents d'un isomère à l'autre, au point que l'un des isomères optiquement actifs peut être plus toxique que l'autre. Un des exemples les plus frappants dans ce domaine est celui de la thalidomide dont les deux énantiomères possèdent des effets hypnotiques similaires, mais dont seul l'énantiomère S manifeste des effets tératogènes.

Enfin, il faut encore ajouter que les isomères optiques constituent des sondes de première importance pour l'étude des interactions chimiques avec les mécanismes physiologiques (par exemple, la sélectivité de fixation sur un récepteur).

C'est la raison pour laquelle de nombreux laboratoires pharmaceutiques consacrent beaucoup d'efforts et de temps pour isoler ou synthétiser les énantiomères d'un composé pharmacologiquement actif et pour en étudier les propriétés thérapeutiques.

Dans le brevet britannique 2.225.321, on décrit un procédé de préparation des énantiomères du dichlorhydrate de l'acide 2-[2-[4-[(4-chlorophényl)phénylméthyl]-1-pipérazinyl]éthoxyl-acétique connu comme médicament antihistaminique non sédatif sous le nom générique de cétirizine. Ce procédé est basé sur l'utilisation de la 1-[(4-chlorophényl)phénylméthyl]pipérazine lévogyre ou dextrogyre comme produit de départ. Dans ce brevet, les énantiomères de la 1-[(4-chlorophényl)phénylméthyl]pipérazine sont obtenus par résolution chimique de la forme racémique

selon des méthodes connues en soi, en particulier, en formant un sel avec un isomère optique de l'acide tartrique convenablement choisi.

Les inconvénients majeurs de ce procédé sont que d'une part, le rendement de l'étape de résolution de la 1-[(4-chlorophényl)phénylméthyl]pipérazine racémique est extrêmement faible (12,7 % seulement) et, d'autre part, la pureté optique des énantiomères dextrogyre et lévogyre ainsi obtenus est insuffisante et ne permet pas de préparer le produit final avec une pureté optique supérieure à 95 %.

Par voie de conséquence, il serait très souhaitable de pouvoir disposer de nouvelles voies pour préparer les énantiomères de la 1-[(4-chlorophényl)phénylméthyl]pipérazine avec une pureté optique améliorée et avec de meilleurs rendements, en sorte de procurer des matières premières excellentes pour produire des isomères optiquement actifs de médicaments utiles ayant eux aussi une très grande pureté optique.

Mais pour atteindre cet objectif, il est nécessaire de trouver des précurseurs ayant déjà la configuration stéréochimique correcte et qui, d'une part peuvent eux-mêmes être préparés de manière aisée et économique, et avec une pureté optique satisfaisante, et d'autre part, peuvent être transformés facilement et avec des rendements élevés en les énantiomères substantiellement optiquement purs de la 1-[(4-chlorophényl)phénylméthyl]pipérazine.

La demanderesse vient présentement de découvrir un nouveau composé, en l'occurrence, la 1-[(4-chlorophényl)phénylméthyl]-4-[(4-méthylphényl)sulfonyl]pipérazine dont les formes lévogyre et dextrogyre répondent parfaitement à cet objectif.

Le brevet américain n° 3406174 décrit des benzylsulfamides, dont la 1-[(4-chlorophényl)phénylméthyl]-4-(N-sulfamoyl)pipérazine présentée comme agent anticonvulsivant.

La présente invention a pour objet, en tant que composés nouveaux, les énantiomères lévogyre et dextrogyre, de la 1-[(4-chlorophényl)phénylméthyl]-4-[(4-méthylphényl)sulfonyl]pipérazine de formule

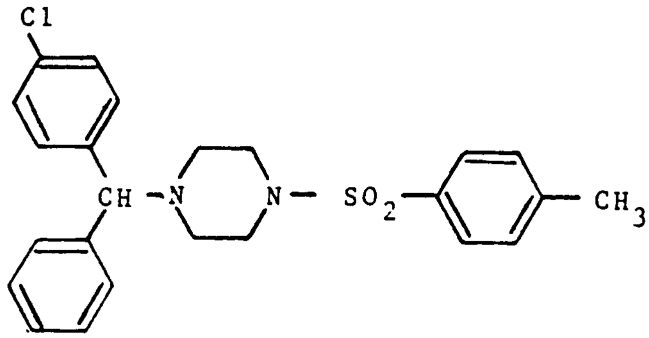

(I)

Conformément à la présente invention, les énantiomères du composé de formule I se présentent avantageusement sous une forme substantiellement optiquement pure.

Dans la présente spécification, par "substantiellement optiquement pur", on entend une pureté optique supérieure à 98 % et cette pureté optique correspond à l'excès, exprimé en pour-cent, de l'isomère optiquement actif présent en quantité majeure par rapport à l'isomère optiquement actif présent en quantité mineure, et est déterminée par chromatographie en phase liquide à haute performance (HPLC) sur une phase stationnaire chirale; elle peut être définie par l'équation décrite à la page 107 de l'ouvrage de J. MARCH, "Advanced Organic Chemistry", John WILEY & Sons, Inc., New York, 3[e] Edition, 1985:

$$\textit{Pureté optique}\ \text{(en \%)} = \frac{[(+)]-[(-)]}{[(+)]+[(-)]} \times 100$$

où

[(+)] = concentration de l'énantiomère dextrogyre, et
[(-)] = concentration de l'énantiomère lévogyre.

La présente invention a également pour objet un procédé de préparation des énantiomères lévogyre et dextrogyre de la 1-[(4-chlorophényl)phénylméthyl]-4-[ (4-méthylphényl)sulfonyl]pipérazine de formule I qui est caractérisé en ce que l'on fait réagir un énantiomère de la (4-chlorophényl)phénylméthylamine de formule

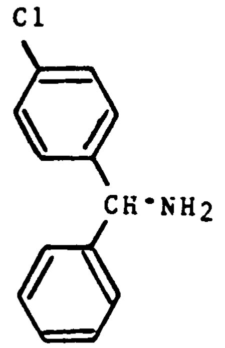

(II)

avec un N,N-diéthyl-4-méthylbenzènesulfonamide de formule

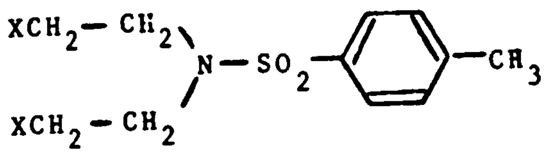

(III)

dans laquelle X représente un atome de chlore, de brome ou d'iode ou le groupe (4-méthylphényl)sulfonyloxy ou méthylsulfonyloxy, en présence de 2,2 à 4,4 équivalents d'une base organique ou minérale par équivalent de l'énantiomère de la (4-chlorophényl)phénylméthylamine et à la température d'ébullition du mélange réactionnel.

Les bases qui conviennent pour préparer les composés de formule I sont soit des bases organiques telles que l'éthyldiisopropylamine, la N-éthylmorpholine, la 2,4,6-triméthylpyridine ou la triéthylamine, de préférence, l'éthyldiisopropylamine, soit des bases inorganiques comme le carbonate de sodium.

Les énantiomères lévogyre et dextrogyre de la (4-chlorophényl)phénylméthylamine de formule II utilisés comme produits de départ sont des composés connus; on peut les préparer par résolution chimique de la (4-chlorophényl)phénylméthylamine racémique au moyen de l'acide tartrique selon des méthodes connues en soi. Ils possèdent une pureté optique d'au moins 98 %.

Les composés de départ de formule III sont aussi des produits connus; ils sont facilement obtenus au départ de la bis(2-hydroxyéthyl)amine par des méthodes connues en soi.

La présente invention a, en outre, pour objet l'utilisation des nouveaux énantiomères lévogyre et dextrogyre de la 1-[(4-chlorophényl)phénylméthyl]-4-[(4-méthylphényl)sulfonyl]pipérazine de formule I pour la préparation des énantiomères substantiellement optiquement purs de la 1-[(4-chlorophényl)phénylméthyl]pipérazine de formule

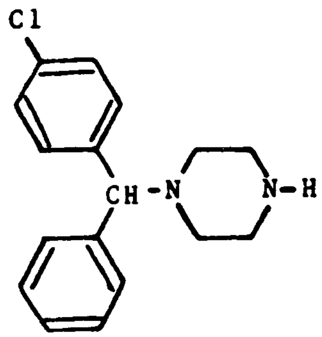

(IV)

Selon la présente invention, on prépare les énantiomères lévogyre et dextrogyre du composé de formule IV par un procédé qui est caractérisé en ce que l'on hydrolyse par l'acide bromhydrique, en milieu acide acétique et en présence d'un composé phénolique, de préférence l'acide 4-hydroxybenzoïque, un énantiomère de la 1-[(4-chlorophényl)phénylméthyl]-4-[(4-méthylphényl)sulfonyl]pipérazine de formule I.

Cette hydrolyse est généralement effectuée à une température comprise entre 18 et 100°C, de préférence à une température voisine de 25°C.

Les avantages obtenus grâce à la mise en oeuvre de la 1-[(4-chlorophényl)phénylméthyl]-4-[(4-méthylphényl)sulfonyl]pipérazine de formule I, sous la forme de ses énantiomères lévogyre et dextrogyre conformes à l'invention, sont multiples.

Ces avantages apparaissent non seulement au niveau de la voie d'accès qui conduit aux énantiomères du composé de formule I, mais aussi au niveau de l'étape de transformation de ces énantiomères pour préparer les énantiomères substantiellement optiquement purs de la 1-[(4-chlorophényl)phénylméthyl]pipérazine de formule IV.

Tout d'abord, la demanderesse a constaté que les énantiomères du composé de formule I, tosylés sur la fonction amine, étaient pratiquement les seuls à pouvoir être synthétisés de manière tout à fait satisfaisante. En effet, si dans la préparation de ces composés, on tente de remplacer le N,N-diéthyl-4-méthylbenzènesulfonamide de formule III par un composé correspondant dans lequel le groupe 4-méthylphénylsulfonyle est remplacé par de l'hydrogène ou par un autre groupement protecteur de la fonction amine, comme par exemple un groupement carbonylé ou alkylé, comme le radical triphénylméthyle, on observe au cours de la formation de l'énantiomère du composé de formule I, une racémisation importante de l'amine de formule II de départ, et/ou du compose de formule I, ou encore, l'apparition de nombreux produits de dégradation.

En outre, les composés de départ de formule III dans lesquels le groupe 4-méthylphénylsulfonyle aurait été remplacé par de l'hydrogène, sont connus pour être extrêmement toxiques du fait de la présence de l'amine libre (moutardes azotées).

Par contre, en utilisant comme produit de départ le N,N-diéthyl-4-méthylbenzènesulfonamide de formule III, on évite ces inconvénients majeurs. En effet, la préparation des énantiomères de la 1-[(4-chlorophényl)phénylméthyl]-4-[(4-méthylphényl)sulfonyl]pipérazine de formule I, conformes à l'invention, se fait selon un procédé non racémisant, avec un rendement élevé, oui peut atteindre 89%, et on les obtient avec une pureté optique supérieure à 98 % qui, dans beaucoup de cas, est voisine de 100 %, au départ de matières premières sulfonées relativement peu toxiques et beaucoup moins dangereuses à manipuler. Ce dernier point représente aussi un avantage non négligeable dans l'optique de l'utilisation industrielle du procédé de l'invention.

D'autre part, l'utilisation des énantiomères du composé de formule I est particulièrement avantageuse pour la préparation des énantiomères de la 1-((4-chlorophényl)phénylméthyl]pipérazine de formule IV. En effet,

- d'une part, les énantiomères de la 1-[(4-chlorophényl)phénylméthyl]pipérazine de formule IV, sont obtenus avec un rendement élevé, bien supérieur à 80 %. Ce rendement est nettement plus élevé que celui du procédé décrit dans le brevet britannique 2.225.321;
- d'autre part, la réaction d'hydrolyse conduisant à la formation des énantiomères du composé de formule IV étant non racémisante, ceux-ci sont obtenus avec une pureté optique très supérieure à 98%, avoisinant même les 100%.

Les énantiomères de la 1-[(4-chlorophényl)phénylméthyl]-4-[(4-méthylphényl)sulfonyl]pipérazine de formule I, conformes à l'invention, ouvrent donc une voie éminemment favorable pour la préparation des énantiomères de la 1-[(4-chlorophényl)phénylméthyl]pipérazine de formule IV

Les énantiomères lévogyre et dextrogyre substantiellement optiquement purs de la 1-[(4-chlorophényl)phénylméthyl]pipérazine de formule IV ainsi préparés trouvent leur intérêt principal comme précurseurs pour la préparation de 1-[(4-chlorophényl)phénylméthyl]pipérazines thérapeutiquement actives de forme lévogyre ou dextrogyre, substantiellement optiquement pures, de formule

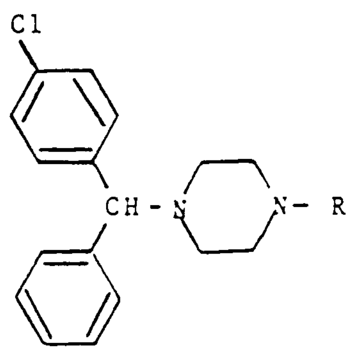

(V)

dans laquelle R représente le radical méthyle, (3-méthylphényl)méthyle, (4-tert-butylphényl)méthyle, 2-(2-hydroxyéthoxy)éthyle, 2-[2-(2-hydoxyéthoxy)éthoxy]éthyle, 2-(carbamoylméthoxy)éthyle, 2-(méthoxycarbonylméthoxy)éthyle et 2-(carboxyméthoxy)éthyle.

Ces composés, déjà connus sous la forme racémique, possèdent des propriétés pharmacologiques intéressantes et peuvent être utilisés pour le traitement de l'asthme, des allergies, de l'inflammation ou comme agents sédatifs, tranquillisants ou anxiolytiques.

Les composés de formule V sous forme racémique dans lesquels R représente le radical 2-(carbamoylméthoxy)éthyle ou 2-(méthoxycarbonylméthoxy)éthyle sont décrits dans le brevet européen 58146. Selon ce document, ces composés sous forme racémique peuvent être préparés en faisant réagir la 1-[(4-chlorophényl)phénylméthyl]pipérazine racémique avec le 2-chloroéthoxyacétamide ou le 2-chloroéthoxyacétate de méthyle, respectivement.

Le composé de formule V sous forme racémique dans lequel R représente le radical 2-(2-hydroxyéthoxy)éthyle est, quant à lui, décrit dans le brevet belge n° 523901. Selon ce document, ce composé sous forme racémique peut être préparé en faisant réagir la 1-[(4-chlorophényl)phénylméthyl]pipérazine racémique avec le 1-chloro-2-(2-hydroxyéthoxy)éthane.

Parmi les composés de formule V sous forme lévogyre ou dextrogyre ayant un intérêt thérapeutique particulier, on citera plus particulièrement les énantiomères lévogyre et dextrogyre de la 1-[(4-chlorophényl)phénylméthyl]-4-méthylpipérazine, de la 1-[(4-chlorophényl)phénylméthyl]-4-[(3-méthylphényl)méthyl]pipérazine, de la 1-[(4-tert-butylphényl)méthyl]-4-[(4-chlorophényl)phénylméthyl]pipérazine, du 2-[2-[4-[(4-chlorophényl)phénylméthyl]-1-pipérazinyl]éthoxy]éthanol, du 2-[2-[2-[4-[(4-chlorophényl)phénylméthyl]-1-pipérazinyl]éthoxy]éthoxy]éthanol, du 2-[2-[4-[(4-chlorophényl)phénylméthyl]-1-pipérazinyl]éthoxy]acétamide, du 2-[2-[4-[(4-chlorophényl)phénylméthyl]-1-pipérazinyl]éthoxy]acétate de méthyle et de l'acide 2-[2-[4-[(4-chlorophényl)phénylméthyl]-1-pipérazinyl]éthoxy]acétique, ainsi que les sels pharmaceutiquement acceptables de ces énantiomères.

La préparation de ces énantiomères substantiellement optiquement purs est effectuée au moyen de méthodes connues en soi et consiste à faire réagir à chaud un énantiomère du composé de formule IV avec un halogénure de formule RX dans laquelle R a la signification donnée plus haut et X représente un atome d'halogène. Les énantiomères de formule V possèdent des propriétés antihistaminiques intéressantes; en particulier, ils présentent une différence très nette de comportement en ce qui concerne l'inhibition du récepteur $H_1$ de l'histamine, l'un des énantiomères étant un inhibiteur compétitif et l'autre non compétitif.

Les essais pharmacologiques décrits ci-après mettent en évidence ces propriétés.

Les exemples qui suivent illustrent l'invention sans toutefois la limiter. Dans ces exemples, les points de fusion ont été déterminés par calorimétrie à balayage différentiel (D.S.C.) avec un gradient de température de 20°C/min. La pureté optique telle que définie précédemment a été déterminée par chromatographie en phase liquide à haute performance, sur une phase stationnaire chirale (colonne CHIRALPAK AD, 250 x 4,6 mm; éluant: mélange 50:50:0,1 (v/v/v) hexane - éthanol - diéthylamine; pression 104 bars; température 25°C; débit 1 ml/min).

Exemple 1. Préparation des énantiomères lévogyre et dextrogyre de la (4-chlorophényl)phénylméthylamine de formule II.

1. (-)-(4-chlorophényl)phénylméthylamine lévogyre.

On prépare ce composé par résolution de la (4-chlorophényl)phénylméthylamine racémique au moyen d'acide (+)-tartrique selon la méthode décrite par R. CLEMO et al. (J. Chem. Soc., (1939), p. 1958-1960).

2. (+)-(4-chlorophényl)phénylméthylamine dextrogyre.

On prépare ce composé par résolution de la (4-chlorophényl)phénylméthylamine racémique au moyen d'acide (-)-tartrique selon la méthode décrite par R. CLEMO et al. (loc. cit.).

3. Récupération de l'énantiomère de la (4-chlorophényl)phénylméthylamine non utilisé.

Dans le but de récupérer et de recycler l'énantiomère de la (4-chlorophényl)phénylméthylamine qui n'est pas utilisé, on soumet ce composé à une réaction de racémisation et on utilise ensuite la (4-chlorophényl)phénylméthylamine racémique ainsi obtenue dans une nouvelle étape de résolution au moyen d'un isomère de l'acide tartrique selon la méthode décrite en 1 ou 2 ci-dessus.

On met en suspension 4,35 g (0,02 mole) de (+)-(4-chlorophényl)phénylméthylamine dextrogyre, 244 mg (0,002 mole) de 2-hydroxybenzaldéhyde et 1,1 g (0,02 mole) de méthylate de sodium dans 21,8 ml de méthanol. On chauffe le mélange à reflux pendant 5 heures et demie puis on laisse revenir à la température ambiante et on ajoute goutte à goutte 6,7 ml d'acide chlorhydrique concentré. On évapore le méthanol, on reprend le résidu avec 50 ml d'eau et on ajoute encore 25 ml d'acide chlorhydrique concentré. Après une heure, on filtre et lave à l'eau le précipité blanc

qui s'est formé et on le sèche sous vide à 40°C. On obtient 3,7 g de (4-chlorophényl)phénylméthylamine racémique. Rendement: 73 %.

$[\alpha]_D^{25}$ : 0° (c=1, méthanol)

Exemple 2. Préparation des N,N-diéthyl-4-méthylbenzènesulfonamides de formule III.

1. 4-méthyl-N,N-bis[2-[(4-méthylphényl)sulfonyloxyléthyl]benzènesulfonamide. (formule III, X= (4-méthylphényl)sulfonyloxy).

Ce composé est préparé à partir de N,N-bis(2-hydroxyéthyl)-4-méthylbenzènesulfonamide selon la méthode décrite par D.H. PEACOCK et U.C. DUTTA (J. Chem. Soc., (1934), p.1303-1305).

P.F.: 75,9°C. Rendement 79,7%.

2. 4-méthyl-N,N-bis[2-(méthylsulfonyloxy)éthyl]benzènesulfonamide. (formule III, X= méthylsulfonyloxy).

On refroidit à 5°C une solution de 11,4 g (0,1 mole) de chlorure de méthanesulfonyle dans 17,1 ml de dichlorométhane. On ajoute alors goutte à goutte et sous agitation, une solution de 13 g (0,05 mole) de N,N-bis(2-hydroxyéthyl)-4-méthylbenzènesulfonamide et de 10,1 g (0,1 mole) de triéthylamine dans 52 ml de dichlorométhane. On laisse le mélange revenir à la température ambiante et on poursuit l'agitation pendant 3 heures. On extrait le mélange réactionnel à trois reprises par 40 ml d'eau. La phase organique est séchée sur sulfate de sodium, filtrée et concentrée dans un évaporateur rotatif. L'huile obtenue cristallise dans l'éthanol. On obtient 17,8 g de 4-méthyl-N,N-bis[2-(méthylsulfonyloxy)éthyl]benzènesulfonamide,

P.F.: 64,6°C. Rendement: 85,7%.

3. N,N-bis(2-chloroéthyl)-4-méthylbenzènesulfonamide. (formule III, X= Cl).

Ce composé est préparé selon la méthode décrite par K.A. AL-RASHOOD et al. (Arzneim.-Forsch./Drug Res. 40(II) (1990), p.1242-1245).

P.F.: 45,8°C. Rendement: 69,0%.

4. N,N-bis(2-iodoéthyl)-4-méthylbenzènesulfonamide. (formule III, X= I).

On dissout 5,7 g (0,01 mole) de 4-méthyl-N,N-bis[2-[(4-méthylphényl)sulfonyloxy]éthyl]benzènesulfonamide (préparé comme indiqué en 1 ci-dessus) dans 57 ml d'acétone et on y ajoute 4,5 g (0,03 mole) d'iodure de sodium. On chauffe le mélange à reflux pendant 22 heures. On laisse refroidir et on évapore l'acétone. On reprend le résidu solide par un mélange de 10 ml d'eau et de 25 ml de dichlorométhane et on sépare les deux phases. On extrait la phase aqueuse avec 25 ml de dichlorométhane et on réunit les phases organiques. On lave cette phase organique successivement avec 10 ml d'une solution aqueuse de thiosulfate de sodium à 10 %, puis avec 10 ml d'eau. On sèche la phase organique sur sulfate de sodium, on filtre et on évapore. Le solide blanc obtenu est séché sous vide à 25°C. On obtient 4,7 g de N,N-bis(2-iodoéthyl)-4-méthylbenzènesulfonamide.

P.F.: 93,8°C. Rendement: 98 %.

5. N,N-bis(2-bromoéthyl)-4-méthylbenzènesulfonamide. (formule III, X= Br).

On prépare ce composé selon la méthode décrite en 4 ci-dessus, mais on remplace l'iodure de sodium par le bromure de sodium. Le mélange réactionnel est chauffé à reflux dans l'acétone pendant 16 jours.

P.F.: 69,2°C. Rendement: 98,7 %.

Exemple 3. Préparation des énantiomères de la 1-[(4-chlorophényl)phénylméthyl]-4-[(4-méthylphényl)sulfonyl]pipérazine de formule I.

A1. (-)-1-[(4-chlorophényl)phénylméthyl]-4-[(4-méthylphényl)sulfonyl]pipérazine lévogyre.

Dans un ballon de 25 ml, on mélange 3,4 g (0,0156 mole) de (-)-(4-chlorophényl)phénylméthylamine lévogyre (préparée à l'exemple 1.1) et 5,1 g (0,0172 mole) de N,N-bis(2-chloroéthyl)-4-méthylbenzènesulfonamide (préparé à l'exemple 2.3) dans 6 ml (4,4 g ou 0,0343 mole) d'éthyldiisopropylamine. On chauffe le mélange à reflux (127°C) pendant 4 heures. Sous agitation, on refroidit ensuite à 86°C et on ajoute en une fois 13,8 ml de méthanol. On refroidit alors le mélange au bain de glace et on maintient l'agitation pendant 1 heure. On filtre le précipité qui s'est formé, on le lave avec 10 ml de méthanol et on le sèche sous vide à 40°C. On recristallise le produit dans un mélange 3:1 (v/v) méthanol - acétone. On obtient 6 g de (-)-1-[(4-chlorophényl)phénylméthyl]-4-[(4-méthylphényl)sulfonyl]pipérazine lévogyre.

P.F.: 171,1°C. Rendement: 87,2 %.
$[\alpha]_D^{25}$ : -40,68° (c=1, toluène)
Pureté optique: 100 %

| Analyse pour $C_{24}H_{25}ClN_2O_2S$ en %: | | | | | |
|---|---|---|---|---|---|
| calculé: | C 65,37 | H 5,71 | N 6,35 | Cl 8,04 | S 7,27 |
| trouvé: | 65,95 | 5,80 | 6,60 | 8,12 | 7,33 |

A2 à A5. Influence de la nature de la base.

On prépare également la (-)-1[(4-chlorophényl)phénylméthyl]-4-[(4-méthylphényl)sulfonyl)pipérazine lévogyre à partir de la N,N-bis(2-chloroéthyl)-4-méthylbenzènesulfonamide en utilisant la méthode décrite en A1 ci-dessus, mais on remplace l'éthyldiisopropylamine par diverses autres bases.

Dans le tableau I, on indique

dans la première colonne, le numéro de l'exemple,
dans la deuxième colonne, la base utilisée,
dans la troisième colonne, la quantité de base utilisée exprimée en équivalents par équivalent de (-)-(4-chlorophényl)phénylméthylamine,
dans la quatrième colonne, le temps (en heures) pendant lequel on a maintenu le mélange réactionnel à reflux,
dans la cinquième colonne, le rendement obtenu en (-)-1-[(4-chlorophényl)phénylméthyl]-4-[(4-méthylphényl)sulfonyl]pipérazine lévogyre, et
dans la sixième colonne, la pureté optique du produit obtenu exprimée en pour-cent.

TABLEAU I

| Exemple 3 | Base | Quantité de base (éq.) | Durée (heures) | Rendement (%) | Pureté Optique (%) |
|---|---|---|---|---|---|
| A1 | éthyldiisopropylamine | 2,3 | 4 | 87,2 | ≈ 100 |
| A2 | 2,4,6-triméthylpyridine | 3,0 | 1,5 | 64,2 | ≈ 100 |
| A3 | N-éthylmorpholine | 2,2 | 4 | 61,2 | 98,4 |
| A4 | triéthylamine | 3,0 | 48 | 59,7 | ≈ 100 |
| A5 | $Na_2CO_3$/xylène(*) | 3,0 | 28 | 56,7 | ≈ 100 |

(*) solvant auxiliaire pour la réaction.

A l'examen de ce tableau, il apparalt que la nature de la base n'a que très peu d'influence sur la pureté optique du produit obtenu. Il apparaît toutefois que l'éthyldiisopropylamine est nettement plus avantageuse du point de vue du rendement de la réaction.

A6 à A9. Influence de la nature du N,N-diéthyl-4-méthylbenzènesulfonamide de formule III .

On prépare également la (-)-1-[(4-chlorophényl)phénylméthyl]-4-[(4-méthylphényl)sulfonyl]pipérazine lévogyre en utilisant la méthode décrite en A1 ci-dessus, mais on remplace le N,N-bis(2-chloroéthyl)-4-méthylbenzènesulfonamide de formule III (X= Cl) utilisé comme produit de départ par le dérivé bromé (X= Br), iodé (X= I), tosylé (X= (4-méthylphényl)sulfonyloxy) et mésylé (X= méthylsulfonyloxy), préparés respectivement aux exemples 2.5, 2.4, 2.1 et 2.2.

Dans le tableau II, on indique

dans la première colonne, le numéro de l'exemple,
dans la deuxième colonne, la nature du substituant X dans le composé de départ de formule III,
dans la troisième colonne, la quantité du composé de formule III utilisée exprimée en équivalents par équivalent de (-)-(4-chlorophényl)phénylméthylamine,
dans la quatrième colonne, le temps, exprimé en heures, pendant lequel on maintient le mélange réactionnel à reflux,
dans la cinquième colonne, le rendement obtenu en (-)-1-[(4-chrorophényl)phénylméthyl]-4-[(4-méthylphényl)sulfonyl]pipérazine lévogyre et
dans la sixième colonne, la pureté optique du produit exprimée en pour-cent.

TABLEAU II

| Exemple 3 | Composé de formule III Substituant X | Quantité de III (éq.) | Durée (heures) | Rendement (%) | Pureté optique (%) |
|---|---|---|---|---|---|
| A1 | Cl | 1,1 | 4 | 87,2 | ≈ 100 |
| A6 | Br | 1 | 1 | 88,9 | ≈ 100 |
| A7 | méthylsulfonyloxy | 1 | 2 | 84,6 | ≈ 100 |
| A8 | I | 1 | 1 | 84,1 | 99,4 |
| A9 | (4-méthylphényl)sulfonyloxy | 1 | 1 | 83,8 | ≈ 100 |

A l'examen de ce tableau, il apparaît que la nature du composé de formule III n'a que très peu d'influence sur la pureté optique du produit obtenu. De plus, on voit qu'il n'influence que très peu le rendement de la réaction, le meilleur rendement étant toutefois obtenu avec le dérivé bromé.

B. (+)-1-[(4-chlorophényl)phénylméthyl]-4-[(4-méthylphényl)sulfonyl]pipérazine dextrogyre.

Dans un ballon à trois cols de 500 ml, on introduit 57 g (0,2618 mole) de (+)-(4-chlorophényl)phénylméthylamine dextrogyre (préparée à l'exemple 1.2) et 86,4 g (0,2917 mole) de N,N-bis(2-chloroéthyl)-4-méthylbenzènesulfonamide (préparé à l'exemple 2.3) dans 200 ml (1,15 mole) d'éthyldiisopropylamine. On chauffe le mélange à reflux pendant 3 heures puis on le verse dans 400 ml de méthanol et on agite le mélange refroidi dans un bain de glace pendant 1 heure. On filtre le précipité qui s'est formé, on le lave avec du méthanol et on sèche sous vide à 50°C. On obtient 88,6 g de (+)-1-[(4-chlorophényl)phénylméthyl]-4-[(4-méthylphényl)sulfonyl]pipérazine dextrogyre.

P.F.: 173,3°C. Rendement: 76,7 %
$[\alpha]_D^{25}$ : +43,2° (c=0,5, toluène)
Pureté optique: 98,35 %

| Analyse pour $C_{24}H_{25}ClN_2O_2S$ en %: | | | | | |
|---|---|---|---|---|---|
| calculé: | C 65,38 | H 5,71 | N 6,35 | Cl 8,04 | S 7,27 |
| trouvé: | 64,98 | 5,70 | 6,40 | 7,96 | 7,35 |

Exemple 4. Préparation des énantiomères lévogyre et dextrogyre de la 1-[(4-chlorophényl)phénylméthyl]pipérazine de formule IV.

1. (-)-1-[(4-chlorophényl)phénylméthyl]pipérazine lévogyre.

Dans 1 litre d'une solution à 30 % d'acide bromhydrique dans de l'acide acétique, on introduit 370 g (0,839 mole) de (-)-1-[(4-chlorophényl)phénylméthyl]-4-[(4-méthylphényl)sulfonyl]pipérazine lévogyre (préparée à l'exemple 3.A1) et 405 g d'acide 4-hydroxybenzoïque. On agite la suspension pendant 17 heures à 25 °C. On ajoute ensuite 2 litres d'eau et on refroidit au bain de glace. Il se forme un précipité que l'on filtre et lave avec 750 ml d'eau. On ajoute au filtrat 2 litres de toluène et 0,9 litre d'une solution aqueuse d'hydroxyde de sodium à 50 %. On décante et on lave la phase organique avec 100 ml d'eau puis encore une fois avec 1 litre d'une solution aqueuse saturée de chlorure de sodium. On sèche la phase organique sur sulfate de sodium, on filtre et on évapore le solvant sous pression réduite. Le résidu est recristallisé à l'ébullition dans 600 ml d'hexane. On filtre à chaud pour éliminer un léger insoluble et on laisse cristalliser le filtrat, d'abord, à la température ambiante, ensuite, par refroidissement au bain de glace pendant 24 heures. On filtre les cristaux, on lave à l'hexane et on sèche sous vide à 40°C. On obtient 204,15 g de (-)-1-[(4-chlorophényl)phénylméthyl]pipérazine lévogyre.

P.F.: 90,5°C. Rendement: 84,8 %.
$[\alpha]_D^{25}$ : -14,25° (c=1, méthanol)
Pureté optique: ≥ 99,8 %

| Analyse pour $C_{17}H_{19}ClN_2$ en %: | | | | |
|---|---|---|---|---|
| calculé: | C 71,19 | H 6,68 | N 9,77 | Cl 12,36 |
| trouvé: | 71,19 | 6,84 | 9,55 | 11,48 |

2. (+)-1-[(4-chlorophényl)phénylméthyl]pipérazine dextrogyre.

On prépare la (+)-1-[(4-chlorophényl)phénylméthyl]pipérazine dextrogyre suivant le procédé décrit en 1 ci-dessus, mais on remplace l'énantiomère lévogyre de la 1-[(4-chlorophényl)phénylméthyl]-4-[(4-méthylphényl)sulfonyl]pipérazine de départ par l'énantiomère dextrogyre (préparé à l'exemple 3.B).

P.F.: 91,5°C. Rendement: 97,9 %
$[\alpha]_D^{25}$ : +14,94° (c=1, méthanol)
Pureté optique: 100 %

| Analyse pour $C_{17}H_{19}ClN_2$ %: | | | | |
|---|---|---|---|---|
| calculé: | C 71,19 | H 6,68 | N 9,77 | Cl 12,36 |
| trouvé: | 70,90 | 6,74 | 9,72 | 12,23 |

Exemple 5. Utilisation des énantiomères de la 1-[(4-chlorophényl)phénylméthyl]pipérazine pour la préparation de composés thérapeutiquement actifs de formule V.

1. Dichlorhydrate lévogyre de la 1-[(4-chlorophényl)phénylméthyl]-4-[(3-méthylphényl)méthyl]pipérazine.

On chauffe à 50°C une solution contenant 10 g (0,0348 mole) de (+)-1-[(4-chlorophényl)phénylméthyl]pipérazine dextrogyre (préparée à l'exemple 4.2) dans 100 ml de n-butanol. On y ajoute 5,5 ml (0,0417 mole) de 1-chlorométhyl-3-méthylbenzène, 8,9 g (0,0836 mole) de carbonate de sodium et 0,5 g (0,0030 mole) d'iodure de potassium. On chauffe le mélange à la température de reflux pendant 3 heures. On refroidit, on élimine les résidus solides par filtration et on les rince avec 200 ml de toluène. On réunit les phases organiques et on évapore les solvants jusqu'à obtention d'une huile résiduelle. On redissout l'huile dans 500 ml d'éthanol auxquels on ajoute 15 ml d'acide chlorhydrique concentré dissous dans 35 ml d'éthanol. On refroidit au bain de glace, on filtre le précipité et le filtrat est évaporé. Le résidu obtenu après évaporation et le précipité sont réunis et remis en suspension dans 100 ml d'alcool isopropylique. On filtre la suspension, le solide est lavé avec un peu d'alcool isopropylique et séché sous vide à 50°C. On obtient 12,7 g de dichlorhydrate lévogyre de la 1-[(4-chlorophényl)phénylméthyl]-4-[(3-méthylphényl)méthyl]pipérazine.

P.F.: 252,3°C. Rendement: 78,6 %
$[\alpha]^{25}_{365}$ : -27,96° (c=1, méthanol)
Pureté optique: ≈ 100 %

| Analyse pour $C_{25}H_{27}ClN_2.2HCl$ en %: | | | | |
|---|---|---|---|---|
| calculé: | C 64,73 | H 6,30 | N 6,04 | $Cl^-$ 15,29 |
| trouvé: | 64,45 | 6,42 | 5,93 | 15,18 |

2. Dichlorhydrate dextrogyre de la 1-[(4-chlorophényl)phénylméthyl]-4-[(3-méthylphényl)méthyl]pipérazine.

Pour préparer le dichlorhydrate dextrogyre de la 1-[(4-chlorophényl)phénylméthyl]-4-[(3-méthylphényl)méthyl]pipérazine, on remplace dans la méthode décrite en 1 ci-dessus, la (+)-1-[(4-chlorophényl)phénylméthyl]pipérazine dextrogyre de départ par l'énantiomère lévogyre (préparé à l'exemple 4.1) en mettant en oeuvre les mêmes quantités de réactifs. On obtient 13 g de dichlorhydrate dextrogyre de la 1-[(4-chlorophényl)phénylméthyl]-4-[(3-méthylphényl)méthyl]pipérazine.

P.F.: 252,9°C. Rendement: 80,4 %.
$[\alpha]^{25}_{365}$ : +27,5° (c=1, méthanol)
Pureté optique: ≈ 100 %

| Analyse pour $C_{25}H_{27}ClN_2.2HCl$ en %: | | | | |
|---|---|---|---|---|
| calculé: | C 64,73 | H 6,30 | N 6,04 | $Cl^-$ 15,29 |
| trouvé: | 64,47 | 6,32 | 5,88 | 15,18 |

3. Dichlorhydrate lévogyre de la 1-[(4-tert-butylphényl)méthyl]-4-[(4-chlorophényl)phénylméthyl]pipérazine.

On chauffe à 50°C une solution contenant 10 g (0,0348 mole) de (+)-1-[(4-chlorophényl)phénylméthyl]pipérazine dextrogyre (préparée à l'exemple 4.2) dans 100 ml de n-butanol. On y ajoute 7,6 ml (0,0418 mole) de 1-chlorométhyl-4-tert-butylbenzène, 8,9 g (0,0836 mole) de carbonate de sodium et 0,5 g (0,0030 mole) d'iodure de potassium. On chauffe le mélange à la température de reflux pendant 1 heure. Ensuite, on refroidit, on élimine les matières solides par filtration et on les rince avec 200 ml de toluène. On réunit les phases organiques et on évapore les solvants jusqu'à

obtention d'une huile résiduelle. On redissout cette huile dans 300 ml d'acétone et on ajoute 15 ml d'acide chlorhydrique concentré dissous dans 35 ml d'acétone, puis on ajoute encore 200 ml d'acétone. On refroidit au bain de glace, puis on filtre le précipité formé et on le sèche sous vide à 50°C. On obtient 14,68 g de dichlorhydrate lévogyre de la 1-[(4-tert-butylphényl)méthyl]-4-[(4-chlorophényl)phénylméthyl]pipérazine.

P.F.: 257,7°C. Rendement: 83,3 %.
$[\alpha]_{365}^{25}$ : -13,26° (c=0,2, méthanol)
Pureté optique: ≈ 100 %

| Analyse pour $C_{28}H_{33}ClN_2.2HCl$ en %: | | | | |
|---|---|---|---|---|
| calculé: | C 66,47 | H 6,97 | N 5,54 | $Cl^-$ 14,01 |
| trouvé: | 66,35 | 7,39 | 5,45 | 13,85 |

4. Dichlorhydrate dextrogyre de la 1-[(4-tert-butylphényl)méthyl]-4-[(4-chlorophényl)phénylméthyl]pipérazine.

Ce composé a été préparé en utilisant la méthode décrite au point 3 ci-dessus pour préparer l'énantiomère lévogyre, en partant toutefois de 4 g de (-)-1-[(4-chlorophényl)phénylméthyl]pipérazine lévogyre (préparée à l'exemple 4.1). On obtient 4,75 g de dichlorhydrate dextrogyre de la 1-[(4-tert-butylphényl)méthyl]-4-[(4-chlorophényl)phénylméthyl]pipérazine.

P.F.: 273,9°C. Rendement: 67,4 %.
$[\alpha]_{365}^{25}$ : +11,33° (c=0,2, méthanol)
Pureté optique: ≈ 100 %

| Analyse pour $C_{28}H_{33}ClN_2.2HCl$ en %: | | | | |
|---|---|---|---|---|
| calculé: | C 66,47 | H 6,97 | N 5,54 | $Cl^-$ 14,01 |
| trouvé: | 66,37 | 7,16 | 5,27 | 13,85 |

5. Dichlorhydrate lévogyre du 2-[2-[4-[(4-chlorophényl)phénylméthyl]-1-pipérazinyl]éthoxyléthanol.

On chauffe à 50°C une solution contenant 10 g (0,0348 mole) de (+)-1-[(4-chlorophényl]phénylméthyl]pipérazine dextrogyre (préparée à l'exemple 4.2) dans 100 ml de n-butanol. On y ajoute 5 ml (0,0464 mole) de 2-(2-chloréthoxy)éthanol, 8,9 g (0,0836 mole) de carbonate de sodium et 0,5 g (0,0030 mole) d'iodure de potassium. On chauffe le mélange à la température de reflux pendant 16 heures. Ensuite, on ajoute encore 2 ml de 2-(2-chloréthoxy)éthanol et on maintient à reflux pendant 4 heures supplémentaires. On refroidit, on filtre et on lave le précipité avec 200 ml de toluène. On évapore les phases organiques jusqu'à obtention d'une huile que l'on dissout dans 100 ml d'éthanol. On ajoute 12 ml d'acide chlorhydrique concentré dissous dans 38 ml d'éthanol. On évapore le solvant et on recristallise le résidu dans l'éthanol. On filtre et on lave le précipité avec un peu d'alcool isopropylique (1$^{er}$ jet). On évapore le filtrat et le résidu solide est lavé avec un peu d'alcool isopropylique (2$^{ème}$ jet). Les deux jets sont recristallisés ensemble dans un mélange 30:1 (v/v) alcool isopropylique - méthanol. On obtient 10,57 g de dichlorhydrate lévogyre du 2-[2-[4-[(4-chlorophényl)phénylméthyl]-1-pipérazinyl]éthoxy]éthanol.

P.F.: 229,8°C. Rendement: 67,8 %.
$[\alpha]_{365}^{25}$ : -6,07° (c=1, eau)
Pureté optique: ≈ 100 %

| Analyse pour $C_{21}H_{27}ClN_2O_2$.2HCl en %: | | | | |
|---|---|---|---|---|
| calculé: | C 56,32 | H 6,53 | N 6,26 | $Cl^-$ 15,83 |
| trouvé: | 56,32 | 6,79 | 6,08 | 15,63 |

6. Dichlorhydrate dextrogyre du 2-[2-[4-[(4-chlorophényl)phénylméthyl]-1-pipérazinyl]éthoxy]éthanol.

En mettant en oeuvre les mêmes quantités de réactifs utilisées dans la méthode décrite au point 5 ci-dessus, on prépare de la même façon l'énantiomère dextrogyre au départ de la (-)-1-[(4-chlorophényl)phénylméthyl]pipérazine lévogyre (préparée à l'exemple 4.1). On obtient 11,7 g de dichlorhydrate dextrogyre du 2-[2-[4-[(4-chlorophényl)phénylméthyl]-1-pipérazinyl]éthoxy]éthanol.

P.F.: 231,3°C. Rendement: 70,5 %
$[\alpha]^{25}_{365}$ : +5,16° (c=1, eau)
Pureté optique: ≈ 100 %

| Analyse pour $C_{21}H_{27}ClN_2O_2$.2HCl en %: | | | | |
|---|---|---|---|---|
| calculé: | C 56,32 | H 6,52 | N 6,25 | $Cl^-$ 15,83 |
| trouvé: | 55,75 | 6,54 | 6,10 | 15,81 |

7. Dichlorhydrate lévogyre du 2-[2-[2-[4-[(4-chlorophényl)phénylméthyl]-1-pipérazinyl]éthoxyléthoxyléthanol.

On chauffe à 40°C une solution contenant 10 g (0,0348 mole) de (+)-1-[(4-chlorophényl)phénylméthyl]pipérazine dextrogyre (préparée à l'exemple 4.2) dans 100 ml de n-butanol. On y ajoute 6,1 ml (0,0419 mole) de 2-[2-(2-chloréthoxy)éthoxy]éthanol, 8,9 g (0,0836 mole) de carbonate de sodium et 0,5 g (0,0030 mole) d'iodure de potassium. Le mélange est chauffé à la température de reflux pendant 6 heures. On refroidit, on élimine les matières solides par filtration et on les lave avec un peu de toluène. On rassemble le filtrat et le solvant de lavage et on évapore les solvants. Le résidu obtenu est repris dans 50 ml de toluène que l'on évapore ensuite. On reprend encore le résidu dans 100 ml de toluène, on lave avec 100 ml d'eau et on évapore la phase organique. L'huile obtenue après évaporation est dissoute dans 100 ml d'alcool isopropylique. On y ajoute une solution contenant 12 ml d'acide chlorhydrique concentré dans 38 ml d'alcool isopropylique. On évapore le solvant. On reprend le résidu solide à chaud dans 150 ml d'alcool isopropylique, on y ajoute 100 ml d'hexane et on chauffe la solution à reflux. On refroidit ensuite au bain de glace. On filtre et lave le précipité avec 50 ml du mélange 1:1 (v/v) alcool isopropylique - hexane, ensuite avec 50 ml d'hexane. Le produit solide ainsi obtenu est séché sous vide à 50°C. On obtient 12,2 g de dichlorhydrate lévogyre du 2-[2-[2-[4-[(4-chlorophényl)phénylméthyl]-1-pipérazinyl]éthoxy]éthoxy]éthanol.

P.F. : 198°C. Rendement: 71,13 %.
$[\alpha]^{25}_{365}$ : -10,7° (c=1, méthanol)
Pureté optique: ≈ 100 %

| Analyse pour $C_{23}H_{31}ClN_2O_3$.2HCl en %: | | | | |
|---|---|---|---|---|
| calculé: | C 56,16 | H 6,76 | N 5,69 | $Cl_{tot}$ 21,62 |
| trouvé: | 56,34 | 7,00 | 5,67 | 21,76 |

8. Dichlorhydrate dextrogyre du 2-[2-[2-[4-[(4-chlorophényl)phénylméthyl] 1-pipérazinyl]éthoxy]éthoxy]éthanol.

Au moyen du même procédé que celui décrit au point 7 ci-dessus, on prépare l'énantiomère dextrogyre à partir de la (-)-1-[(4-chlorophényl)phénylméthyl]pipérazine lévogyre (préparée à l'exemple 4.1).

P.F.: 196,1°C. Rendement: 73,8 %.
$[\alpha]_{365}^{25}$ : +8,94° (c=1, méthanol)
Pureté optique: ≈ 100 %

| Analyse pour $C_{23}H_{31}ClN_2O_3.2HCl$ en %: | | | | |
|---|---|---|---|---|
| calculé: | C 56,16 | H 6,76 | N 5,69 | $Cl_{tot}$ 21,62 |
| trouvé: | 56,48 | 6,96 | 5,65 | 22,1 |

9. (-)-2-[2-[4-[(4-chlorophényl)phénylméthyl]-1-pipérazinyl] éthoxy]acétamide lévogyre.

On introduit 77 g (0,2685 mole) de (-)-1-[(4-chlorophényl)phénylméthyl]pipérazine lévogyre (préparée à l'exemple 4.1), 40,5 g (0,2932 mole) de 2-(2-chloréthoxy)acétamide, 62,8 g (0,591 mole) de carbonate de sodium et 2 g (0,0120 mole) d'iodure de potassium dans 700 ml de toluène. On chauffe le mélange à la température de reflux pendant 24 heures. On ajoute alors 10 g de Norit et on filtre à chaud sur Dicalite. On lave le filtrat avec 500 ml d'eau puis avec 500 ml d'une solution aqueuse saturée de chlorure de sodium. On sépare et on sèche la phase organique sur 250 g de sulfate de sodium. On filtre et on évapore le solvant. On reprend l'huile résiduelle à chaud dans 1500 ml d'oxyde diisopropylique. On chauffe la solution à reflux et on laisse cristalliser par refroidissement au bain de glace. On filtre les cristaux, on les lave avec un peu d'oxyde diisopropylique et on sèche sous vide à 40°C. On obtient 82,91 g de (-)-2-[2-[4-[(4-chlorophényl)phénylméthyl]-1-pipérazinyl]éthoxy]acétamide lévogyre.

P.F.: 94,3°C. Rendement: 79,6 %.
$[\alpha]_{365}^{25}$ : -23,5° (c=1, méthanol)
Pureté optique: ≈ 100 %

| Analyse pour $C_{21}H_{26}ClN_3O_2$ en %: | | | | |
|---|---|---|---|---|
| calculé: | C 65,02 | H 6,76 | N 10,83 | Cl 9,14 |
| trouvé: | 65,39 | 6,70 | 10,99 | 9,23 |

10. (+)-2-[2-[4-[(4-chlorophényl)phénylméthyl]-1-pipérazinyl]éthoxy]acétamide dextrogyre.

On introduit 15 g (0,0523 mole) de (+)-1-[(4-chlorophényl)phénylméthyl]pipérazine dextrogyre (préparée à l'exemple 4.2), 8,3 g (0,0601 mole) de 2-(2-chloréthoxy)acétamide, 12,8 g (0,1203 mole) de carbonate de sodium et 0,5 g (0,0030 mole) d'iodure de potassium dans un mélange de 100 ml de p-xylène et de 150 ml de toluène. On chauffe le mélange à la température de reflux pendant 17 heures. On ajoute un peu de Norit et on filtre à chaud sur Dicalite. On rince le résidu sur le filtre avec un peu toluène et on rassemble le filtrat et la solution de lavage. On évapore les solvants et on reprend le résidu dans 100 ml de toluène. On lave la phase organique successivement avec 100 ml d'eau et deux fois avec 100 ml d'une solution aqueuse saturée de chlorure de sodium. On sépare la phase organique et on évapore le solvant. A ce stade, le résidu brut obtenu peut être purifié d'une manière analogue à celle décrite au point 9 ci-dessus, pour obtenir le (+)-2-[2-[4-[(4-chlorophényl)phénylméthyl]-1-pipérazinyl]éthoxy]acétamide dextrogyre sous la forme de la base libre. Toutefois, si on le désire, le résidu brut peut aussi être transformé en dichlorhydrate correspondant de la manière suivante: le résidu brut obtenu est repris dans 100 ml d'acétone, on refroidit au bain de glace et on ajoute goutte à goutte 15 ml d'acide chlorhydrique concentré. Ensuite, on ajoute encore 200 ml d'acétone et on agite le mélange refroidi au bain de glace pendant 1 heure. On filtre le précipité et on sèche sous vide à 50°C. On obtient 19 g de dichlorhydrate lévogyre du 2-[2-[4-[(4-chlorophényl)phénylméthyl]-1-pipérazinyl]éthoxy]acétamide .

P.F.: 237,4°C. Rendement: 78,8 %.
$[\alpha]^{25}_{365}$ : -19,64° (c=1, méthanol).
Pureté optique: ≈ 100 %

| Analyse pour $C_{21}H_{26}ClN_3O_2.2HCl$ en %: | | | | | |
|---|---|---|---|---|---|
| calculé: | C 54,73 | H 6,12 | N 9,12 | $Cl_{tot}$ 23,08 | $Cl^-$ 15,38 |
| trouvé: | 53,70 | 6,20 | 8,91 | 23,08 | 15,61 |

11. Dimaléate lévogyre du 2-[2-[4-[(4-chlorophényl)phénylméthyl]-1-pipérazinyl]éthoxy]acétate de méthyle.

On met en suspension 46 g (0,16 mole) de (-)-1-[(4-chlorophényl)phénylméthyl]pipérazine lévogyre (préparée à l'exemple 4.1), 36,6 g (0,24 mole) de (2-chloréthoxy)acétate de méthyle, 37,3 g (0,35 mole) de carbonate de sodium anhydre et 1,05 g (0,0064 mole) d'iodure de potassium dans 46 ml de toluène. On chauffe la suspension sous agitation à la température de reflux pendant 18 heures. Puis, on refroidit à la température ambiante et on filtre la suspension. On lave les matières solides par 100 ml de toluène et on rassemble le filtrat et la solution de lavage. On évapore le toluène à l'évaporateur rotatif à 50°C et sous pression réduite. On obtient 76 g d'une huile brune que l'on reprend dans 80 ml de dichlorométhane. On purifie la solution par chromatographie (colonne de silice (15 à 40 µm) 1 kg; éluant: dichlorométhane pur progressivement additionné de méthanol jusqu'à un maximum de 2 % de méthanol (v/v)). On obtient ainsi 43,5 g de 2-[2-[4-[(4-chlorophényl)phénylméthyl]-1-pipérazinyl]éthoxylacétate de méthyle, sous forme d'une huile. Rendement: 67,5 %.
Ce composé peut être transformé en le dimaléate correspondant de la manière suivante: on dissout 15 g (0,037 mole) de 2-[2-[4-[(4-chlorophényl)phénylméthyl]-1-pipérazinyl]éthoxylacétate de méthyle préparée ci-dessus dans 45 ml de méthanol à la température de reflux et on y ajoute en une fois 9,1 g (0,078 mole) d'acide maléique. On maintient le mélange à la température de reflux jusqu'à ce que l'acide maléique soit complètement dissous, puis on laisse la solution revenir à température ambiante, toujours sous agitation. On filtre les cristaux qui se sont formés et on les met en suspension dans 15 ml de méthanol. On agite la suspension pendant une heure et demie à la température ambiante puis, encore une fois pendant une heure et demie, à 0 °C. On filtre les cristaux, on les lave par 15 ml de méthanol à 0°C et on les sèche jusqu'à poids constant. On obtient 19,5 g de dimaléate lévogyre du 2-[2-[4-[(4-chlorophényl)phénylméthyl]-1-pipérazinyl]éthoxy]acétate de méthyle.

P.F.: 143,5°C. Rendement: 56 %.
$[\alpha]^{25}_{365}$ : -10,09° (c=1, méthanol).
Pureté optique : ≈ 100 %

| Analyse pour $C_{22}H_{27}ClN_2O_3.2C_4H_4O_4$ en %: | | | |
|---|---|---|---|
| calculé: | C 56,79 | H 5,56 | N 4,41 |
| trouvé: | 56,81 | 5,68 | 4,12 |

12. Dimaléate dextrogyre du 2-[2-[4-[(4-chlorophényl)phénylméthyl]-1-pipérazinyl]éthoxy]acétate de méthyle.

On met en suspension 14,3 g (0,05 mole) de (+)-1-[(4-chlorophényl)phénylméthyl]pipérazine dextrogyre (préparée à l'exemple 4.2), 8,4 g (0,055 mole) de (2-chloréthoxy)acétate de méthyle, 11,7 g (0,11 mole) de carbonate de sodium anhydre et 0,332 g (0,002 mole) d'iodure de potassium dans 14,3 ml de toluène. On chauffe la suspension sous agitation à la température de reflux pendant 17 heures. On ajoute encore 1,52 g (0,01 mole) de (2-chloréthoxy)acétate de méthyle et on chauffe la suspension encore sous agitation à la température de reflux pendant 3 heures. Puis, on refroidit à la température ambiante et on filtre la suspension. On lave les matières solides avec 50 ml de toluène et on rassemble le filtrat et la solution de lavage. On évapore le toluène à l'évaporateur rotatif à 50°C et sous pression réduite. On obtient 22,8 g d'une huile brune que l'on reprend dans 45 ml de dichlorométhane. On purifie la solution par chromatographie (colonne de silice (15 à 40 µm, 1 kg); éluant: dichlorométhane pur progressivement additionné de méthanol jusqu'à un maximum de 2 % de méthanol (v/v)). On obtient 11,1 g de 2-[2-[4-[(4-chlorophényl)phénylméthyl]-1-pipérazinyl]éthoxy]acétate de méthyle sous forme d'une huile. Rendement: 55,1 %.

Ce composé peut être transformé en le dimaléate correspondant de la manière suivante: on dissout 8 g (0,0198 mole) de 2-[2-[4-[(4-chlorophényl)phénylméthyl]-1-pipérazinyl]éthoxy]acétate de méthyle préparé ci-dessus dans 16 ml de méthanol à la température de reflux et on y ajoute en une fois 4,85 g (0,0417 mole) d'acide maléique. On maintient le mélange à la température de reflux jusqu'à ce que l'acide maléique soit complètement dissous, puis on laisse la solution revenir à température ambiante, toujours sous agitation. On filtre les cristaux qui se sont formés et on les met en suspension dans 16 ml de méthanol. On agite la suspension pendant deux heures à la température ambiante. On filtre les cristaux, on les lave par 10 ml de méthanol et on les sèche jusqu'à poids constant. On obtient 7,3 g de dimaléate dextrogyre du 2-[2-[4-[(4-chlorophényl)phénylméthyl]-1-pipérazinyl]éthoxy]acétate de méthyle.

P.F.: 143,2°C. Rendement: 32 %.
$[\alpha]_{365}^{25}$ : + 9,8° (c=1, méthanol).
Pureté optique : ≈ 100 %

| Analyse pour $C_{22}H_{27}ClN_2O_3.2C_4H_4O_4$ en %: | | | |
|---|---|---|---|
| calculé: | C 56,79 | H 5,56 | N 4,41 |
| trouvé: | 56,71 | 5,58 | 4,17 |

13. Dichlorhydrate lévogyre de l'acide 2-[2-[4-[(4-chlorophényl)phénylméthyl]-1-pipérazinyl]éthoxy]acétique.

A une suspension de 25,2 g (0,065 mole) de (+)-2-[2-[4-[(4-chlorophényl)phénylméthyl]-1-pipérazinyl]éthoxy]acétamide dextrogyre (préparé au point 10 ci-dessus), dans 70 ml d'eau, on ajoute goutte à goutte 26 ml d'acide chlorhydrique concentré. La température du mélange s'élève d'elle même à 38 °C. On chauffe ensuite le mélange à 50°C pendant 17 heures. Ensuite, on refroidit le mélange réactionnel au bain de glace et on amène le pH à une valeur comprise entre 4 et 5 par addition d'une solution d'hydroxyde de sodium 4 N. On extrait la solution successivement par 100 ml, puis deux fois par 50 ml de dichlorométhane. Les phases organiques sont réunies et séchées sur sulfate de magnésium. On filtre et on évapore le solvant. On dissout l'huile résiduelle dans 243 ml d'acétone, on traite la solution avec 3,5 g de Norit et on filtre sur Célite. On rince avec 35 ml d'acétone. On chauffe la solution à la température de reflux et on y ajoute goutte à goutte 198 ml (0,13 mole) d'acide chlorhydrique concentré. On refroidit au bain de glace et on laisse le mélange reposer pendant 1 heure. On filtre le précipité qui s'est formé, on le rince avec 100 ml d'acétone et on le sèche sous vide à 50°C. On obtient 24,1 g de dichlorhydrate lévogyre de l'acide 2-[2-[4-[(4-chlorophényl)phénylméthyl]-1-pipérazinyl]éthoxy]acétique.

P.F.: 229,3°C. Rendement: 80,3 %.
$[\alpha]_{D}^{25}$ : -12,79° (c=1, eau).
Pureté optique: ≈ 100 %

| Analyse pour $C_{21}H_{25}ClN_2O_3.2HCl$ en %: | | | | | |
|---|---|---|---|---|---|
| calculé: | C 54,61 | H 5,90 | N 6,07 | $Cl^-$ 15,35 | $Cl_{tot}$ 23,03 |
| trouvé: | 54,67 | 5,91 | 6,03 | 15,34 | 23,28 |

14. Dichlorhydrate dextrogyre de l'acide 2-[2-[4-[(4-chlorophényl)phénylméthy l]-1-pipérazinyl]éthoxy]acétique.

On prépare le dichlorhydrate dextrogyre de l'acide 2-[2-[4-[(4-chlorophényl)phénylméthyl]-1-pipérazinyl]éthoxy]acétique selon la méthode décrite au point 13 ci-dessus, en utilisant 25,2 g (0,065 mole) de (-)-2-[2-[4-[(4-chlorophényl)phénylméthyl]-1-pipérazinyl]éthoxy]acétamide lévogyre (préparé au point 9 ci-dessus). On obtient ainsi 25,6 g du produit désiré.

P.F.: 227,9°C. Rendement: 85,3 %.
$[\alpha]_{365}^{25}$ : +12,87° (c=1, eau).

Pureté optique: 99,87 %

| Analyse pour $C_{21}H_{25}ClN_2O_3.2HCl$ en %: | | | | | |
|---|---|---|---|---|---|
| calculé: | C 54,61 | H 5,90 | N 6,07 | $Cl^-$ 15,35 | $Cl_{tot}$ 23,03 |
| trouvé: | 54,71 | 5,92 | 6,04 | 15,34 | 23,19 |

15. Dichlorhydrate dextrogyre de l'acide 2-[2-[4-[(4- chlorophényl)phénylméthyl]-1-pipérazinyl]éthoxy]acétique.

On introduit sous agitation, à la température ambiante, 13,75 g (0,00216 mole) de dimaléate lévogyre du 2-[2-[4-[(4-chlorophényl)phénylméthyl]-1-pipérazinyl]éthoxy]acétate de méthyle (préparé au point 11 ci-dessus), dans 54 ml d'une solution aqueuse d'hydroxyde de sodium 2N. On extrait le mélange réactionnel successivement avec 100 ml et 75 ml de diéthyléther et on réunit les phases organiques. On sèche cette phase organique sur sulfate de sodium anhydre, on filtre et on lave le résidu de filtration par 50 ml de diéthyléther. On réunit les phases organiques et on évapore le diéthyléther. On reprend l'huile (8,4 g) ainsi obtenue dans 50 ml d'éthanol et on ajoute 1,3 g (0,0229 mole) d'hydroxyde de potassium solide. On chauffe le mélange à la température de reflux pendant une heure, puis on laisse revenir à la température ambiante. On filtre et on évapore le filtrat. Le résidu est repris dans 50 ml d'eau et on concentre à l'évaporateur rotatif afin d'éliminer l'éthanol résiduaire. On ajoute 10 ml d'eau à la solution partiellement concentrée et on amène le pH de la solution à une valeur comprise entre 4 et 5 par addition d'une solution aqueuse d'acide chlorhydrique à 10 %. On extrait la solution par 50 ml de dichlorométhane, on amène à nouveau le pH de la solution à une valeur comprise entre 4 et 5 par addition d'une solution aqueuse d'acide chlorhydrique à 10 % et on l'extrait encore une fois par 50 ml de dichlorométhane. Les phases organiques sont réunies et séchées sur sulfate de magnésium anhydre. On filtre et on évapore le dichlorométhane. On dissout l'huile visqueuse ainsi obtenue (9,8 g) dans 68,6 ml d'acétone, on traite la solution légèrement trouble avec 1 g de charbon actif et on filtre à chaud sur terre à diatomées. On ajoute à la solution jaune limpide chaude ainsi obtenue 3,6 ml (0,043 mole) d'acide chlorhydrique concentré. On laisse revenir la suspension à la température ambiante, sous agitation, puis on agite encore celle-ci pendant une heure à 0 °C. On filtre le précipité qui s'est formé, on le lave par 50 ml d'acétone et on le sèche sous vide à 40°C. On obtient ainsi 6,8 g de dichlorhydrate dextrogyre de l'acide 2-[2-[4-[(4-chlorophényl)phénylméthyl]-1-pipérazinyl] éthoxy]acétique.

P.F.: 227,8°C. Rendement: 70,8 %.
$[\alpha]_{365}^{25}$ : +13,7° (c=1, eau).
Pureté optique: ≈ 100 %

| Analyse pour $C_{21}H_{25}ClN_2O_3.2HCl$ en %: | | | |
|---|---|---|---|
| calculé: | C 54,61 | H 5,90 | N 6,07 |
| trouvé: | 54,18 | 6,02 | 5,68 |

Les composés suivants ont été soumis à des essais pharmacologiques dont les résultats sont reproduits ci-après:

- (-)-1-[(4-chlorophényl)phénylméthyl]pipérazine (composé A, préparé à l'exemple 4.1);
- (+)-1-[(4-chlorophényl)phénylméthyl]pipérazine (composé B, préparé à l'exemple 4.2);
- dichlorhydrate lévogyre de la 1-[(4-chlorophényl)phénylméthyl]-4-[(3-méthylphényl)méthyl]pipérazine (composé C, préparé à l'exemple 5.1);
- dichlorhydrate dextrogyre de la 1-[(4-chlorophényl)phénylméthyl]-4-[(3-méthylphényl)méthyl]pipérazine (composé D, préparé à l'exemple 5.2);
- dichlorhydrate lévogyre de la 1-[(4-tert-butylphényl)méthyl]-4-[(4-chlorophényl)phénylméthyl]pipérazine (composé E, préparé à l'exemple 5.3);
- dichlorhydrate dextrogyre de la 1-[(4-tert-butylphényl)méthyl]-4-[(4-chlorophényl)phénylméthyl]pipérazine (composé F, préparé à l'exemple 5.4);
- dichlorhydrate lévogyre du 2-[2-[4-[(4-chlorophényl)phénylméthyl]-1-pipérazinyl]éthoxy]éthanol (composé G, préparé à l'exemple 5.5);

- dichlorhydrate dextrogyre du 2-[2-[4-[(4-chlorophényl)phénylméthyl]-1-pipérazinyl]éthoxy]éthanol (composé H, préparé à l'exemple 5.6);
- dichlorhydrate lévogyre du 2-[2-[2-[4-[(4-chlorophényl)phénylméthyl]-1-pipérazinyl]éthoxy]éthoxy]éthanol (composé I, préparé à l'exemple 5.7);
- dichlorhydrate dextrogyre du 2-[2-[2-[4-[(4-chlorophényl) phénylméthyl]-1-pipérazinyl]éthoxy]éthoxy]éthanol (composé J, préparé à l'exemple 5.8);
- (-)-2-[2-[4-[(4-chlorophényl)phénylméthyl]-1-pipérazinyl]éthoxy]-acétamide (composé K, préparé à l'exemple 5.9);
- (+)-2-[2-[4-[(4-chlorophényl)phénylméthyl]-1-pipérazinyl]éthoxy]-acétamide (composé L, préparé à l'exemple 5.10);
- dimaléate lévogyre du 2-[2-[4-[(4-chlorophényl)phénylméthyl]-1-pipérazinyl]éthoxy]acétate de méthyle (composé M, préparé à l'exemple 5.11);
- dimaléate dextrogyre du 2-[2-[4-[(4-chlorophényl)phénylméthyl]-1-pipérazinyl]éthoxy]acétate de méthyle (composé N, préparé à l'exemple 5.12);
- dichlorhydrate lévogyre de l'acide 2-[2-[4-[(4-chlorophényl)phénylméthyl]-1-pipérazinyl]éthoxy]-acétique (composé O, préparé à l'exemple 5.13) et
- dichlorhydrate dextrogyre de l'acide 2-[2-[4-[(4-chlorophényl)phénylméthyl]-1-pipérazinyl]éthoxyl-acétique (composé P, préparé à l'exemple 5.14).

1. Affinité vis-à-vis du récepteur $H_1$ de l'histamine.

L'affinité des composés pour le récepteur $H_1$ de l'histamine du cortex de rat a été déterminée au moyen de la méthode décrite par M.M. BILLAH et coll., J. Pharmacol. Exp. Ther., <u>252</u> (3), (1990), 1090-1096.

Ces expériences classiques mettent en jeu la compétition de la fixation sur le récepteur $H_1$ de l'histamine d'une part, du composé à étudier et d'autre part d'un radioligand qui, dans le cas particulier du récepteur $H_1$ de l'histamine est la $[^3H]$mépyramine, connue pour être un antagoniste sélectif de ce récepteur.

Les courbes de déplacement de la fixation de la $[^3H]$mépyramine sont déterminées avec différentes concentrations des composés à étudier, comprises entre $10^{-10}$ et $10^{-4}$ mole/l, et avec 4,5 $10^{-9}$ mole/l de $[^3H]$mépyramine (24,8 Ci/mmole, fournie par New England Nuclear, Belgium).

Des cortex cérébraux de rats mâles Sprague-Dawley sont homogénéisés dans 2 ml par cortex d'un tampon Tris-HCl 20 mM (pH 7,4) contenant du sucrose 250 mM.

On centrifuge les homogénats à 30.000 g durant 30 minutes, à 4°C. Les culots de centrifugation sont remis en suspension dans le même tampon frais et conservés dans l'azote liquide.

Pour déterminer la fixation sur le récepteur $H_1$, on incube les échantillons contenant 0,5 mg de protéine de membrane de cortex, dans 0,5 ml de tampon Tris-HCl 50 mM (pH 7,4) contenant du chlorure de magnésium 2 mM à 25°C pendant 60 minutes en présence de la $[^3H]$mépyramine et du composé à étudier. On sépare la $[^3H]$mépyramine fixée du radioligand libre par filtration rapide sur filtre Whatman GF/C préalablement imprégné pendant au moins 2 heures d'une solution à 0,1 % de polyéthylèneimine, afin de réduire les possibilités de fixation non spécifique du radioligand avec d'autres protéines. On lave ensuite le résidu de filtration à quatre reprises par 2 ml de tampon Tris-HCl 50 mM (pH 7,4) refroidi au bain de glace. La radioactivité est mesurée au moyen d'un compteur à scintillation de particules β Tri-carb 1090 (Camberra-Packard, Belgium) . La fixation non spécifique a été estimée en présence d'une solution aqueuse 10 μM de cétirizine et représente 30 % de la fixation totale. Les valeurs de $IC_{50}$ des composés étudiés (concentrations en mole/l nécessaires pour inhiber de 50% la fixation du radioligand sur le récepteur $H_1$) sont déterminées par analyse des courbes de fixation compétitive (A. DE LEAN et coll., Mol. Pharmacol., <u>21</u> (1982), 5-16) et les valeurs des constantes d'inhibition ($K_i$) ont été calculées au moyen de l'équation de CHENG et PRUSOFF (Y.C. CHENG et W.H. PRUSOFF, Biochem. Pharmacol., <u>22</u> (1973), 3099-3108).

Le tableau III ci-dessous donne les valeurs des $pK_i$ (cologarithme de $K_i$) calculées à partir des $K_i$ (valeur moyenne ± écart par rapport à la moyenne (n=2)).

TABLEAU III

| Composé | $pK_i$ |
|---|---|
| C | 6,2 ± 0,1 |
| D | 7,2 ± 0,2 |
| E | 5,9 ± 0,2 |
| F | 6,2 ± 0,0 |

TABLEAU III (suite)

| Composé | $pK_i$ |
|---|---|
| G | 7,6 ± 0,1 |
| H | 8,7 ± 0,0 |
| I | 7,1 ± 0,0 |
| J | 8,6 ± 0,0 |
| K | 8,6 ± 0,1 |
| L | 6,8 ± 0,1 |
| O | 7,4 ± 0,0 |
| P | 8,2 ± 0,0 |

Il ressort de ce tableau que les composés de formule V possèdent une bonne activité antihistaminique. Ces résultats montrent également qu'il existe entre les énantiomères d'un même composé une différence de $pK_i$ qui correspond à une différence d'affinité relative (donc de $K_i$) d'un facteur compris entre environ 2 et 64 pour le récepteur $H_1$ du cortex de rat. Une telle différence indique que l'on peut utiliser spécifiquement l'énantiomère qui possède l'affinité la plus grande pour ce type de récepteur (par exemple le composé J par rapport à son énantiomère I) comme agent anxiolytique ou tranquillisant pour le traitement de pathologies qui relèvent d'une excitation du système nerveux central.

2. Propriétés antihistaminiques périphériques.

Les propriétés antihistaminiques périphériques des composés sont mises en évidence par la mesure de l'inhibition de la contraction de la trachée isolée de cobaye provoquée par l'histamine selon la méthode décrite par M.H. AMIRI et G. GABELLA (Anat. Embryol., <u>178</u> (1988), 389-397).

On prélève des trachées de cobayes Dunkin-Hartley des deux sexes (poids: 250-500 g), et on les coupe en quatre fragments de 3 segments de cartilage chacun. Ces fragments sont plongés dans une solution de Krebs-Heinseleit à 37°C contenant $10^{-7}$ mole/l d'atropine et $10^{-5}$ mole/l d'indométhacine et sont tendus avec un poids de 1 g. La solution est aérée par passage d'un courant d'oxygène contenant 5 % de dioxyde de carbone. Chaque modification de la tension est enregistrée avec un indicateur de force isométrique K30 (de la société Hugo Sachs Elektronik) couplé à un amplificateur et à un enregistreur Sanborn 7700 (de la société Hewlett Packard). On laisse la préparation ainsi obtenue se stabiliser pendant 1 heure durant laquelle la ligne de base de la tension est réajustée si nécessaire.

Chaque préparation est précontractée par addition dans le milieu de $10^{-4}$ mole/l d'histamine; la contraction observée est prise pour référence (100%). Après lavage et stabilisation, on trace, à titre de contrôle, une courbe cumulative des effets de l'histamine en fonction de sa concentration ($10^{-6}$ $10^{-5}$ et $10^{-4}$ mole/l).

Sur une même préparation, on enregistre encore quatre autres courbes cumulatives des effets de l'histamine en fonction de sa concentration, pour quatre concentrations croissantes des composés à tester.

Les composés à tester sont incorporés dans la préparation 5 minutes avant l'histamine. Entre chaque mesure, on lave les préparations au moins à quatre reprises avec un intervalle de 5 minutes entre chaque lavage. Chaque composé est testé sur au moins 6 fragments de trachée. Lors du tracé de la dernière courbe, on ajoute dans le milieu des concentrations supplémentaires d'histamine de 3,2 $10^{-4}$ et de $10^{-3}$ mole/l afin de déterminer si l'antagonisme est de nature compétitive ou non.

Lorsqu'une inhibition non compétitive est observée, on calcule $pD_2$,, c'est-à-dire le cologarithme de la concentration du composé testé qui provoque une inhibition de 50% de la contraction maximale enregistrée (J.M. VAN ROSSUM, Arch. Int. Pharmacodyn., <u>143</u> (1963), 299-330). Lorsqu'une inhibition compétitive est observée, on calcule le $pA_2$, c'est-à-dire le cologarithme de la concentration du composé testé qui nécessite de doubler la dose d'histamine nécessaire pour obtenir le même effet de contraction.

Le tableau IV ci-dessous donne les $pA_2$ ou $pD_2$, calculés (valeur moyenne ± déviation standard) pour les composés testés.

TABLEAU IV

| Composé | $pA_2$ | $pD_2$, |
|---|---|---|
| A | 5,7 ± 0,4 | - |

TABLEAU IV (suite)

| Composé | $pA_2$ | $pD_2$, |
|---|---|---|
| B | 5,0 ± 0,1 | - |
| G | 6,5 ± 0,3 | - |
| H | - | 6,7 ± 0,1 |
| I | 6,5 ± 0,4 | - |
| J | - | 6,0 ± 0,3 |
| K | - | 6,3 ± 0,2 |
| L | 6,4 ± 0,2 | - |
| O | 6,6 ± 0,3 | - |
| P | - | 6,3 ± 0,2 |

Ce test met en évidence une caractéristique surprenante des couples d'énantiomères lévogyre et dextrogyre testés. A l'exception du couple d'énantiomères A et B, on constate que pour tous les autres couples, un énantiomère est un inhibiteur compétitif, tandis que l'autre est un inhibiteur non compétitif d'où l'intérêt de préparer des dérivés optiquement purs de la 1-[(4-chlorophényl)phénylméthyl]pipérazine.

Les inhibiteurs compétitifs trouvent leur intérêt dans le fait qu'ils ont en général une moindre affinité pour le récepteur $H_1$ de l'histamine du cortex de rat, ce qui laisse présager que les propriétés antiallergiques de ces composés ne sont pas ou peu associées à des effets indésirables sur le système nerveux central, tels que la sédation ou la somnolence par exemple.

Les inhibiteurs non compétitifs présentent l'avantage d'être à même d'inhiber les effets de l'histamine, même lorsque celle-ci est présente à de fortes concentrations locales. Ils sont donc mieux indiqués dans le traitement topique des pathologies de la peau ou des muqueuses.

3. Inhibition de la réaction cutanée induite par l'histamine chez le chien.

Le chien est considéré parmi les espèces animales comme étant celle qui présente une sensibilité à l'histamine relativement proche de celle de l'homme. On considère donc que l'activité antihistaminique d'un composé, observée chez le chien, est prédictive de celle qui serait obtenue chez l'homme.

Dans ce test, on utilise 9 chiens Beagle d'un poids moyen de 12,6 kg et âgés d'environ 2 ans dont l'abdomen a été rasé localement. Dans la zone ainsi rasée, on injecte par voie intradermique, 50 µl d'une solution aqueuse de chlorure de sodium à 0,9 % contenant 10 µg/ml d'histamine. Simultanément, on injecte par voie intraveineuse à la dose de 0,1 ml/kg, une solution de colorant bleu Evans (60 mg/ml dans une solution aqueuse de chlorure de sodium à 0,9 %). Une réaction allergique se développe à l'endroit de l'injection intradermique et on voit apparaître une papule dont on mesure la surface exactement 30 minutes après les deux injections. Cette surface est prise comme surface de référence (100 %).

On administre ensuite le composé à tester par voie orale, à la dose de 0,15 mg/kg (0,32 $10^{-6}$ mole/kg).

Après 0,5, 1,5, 3, 6, 9, 12, 24 et 32 heures à compter de l'administration du composé à tester, on induit de nouvelles papules à différents endroits de l'abdomen par injection d'histamine. A chaque fois, on mesure la surface de la papule induite 30 minutes après l'injection de l'histamine.

L'activité antihistaminique d'un composé sur la réaction allergique cutanée est déterminée par la mesure de la diminution de la surface des papules induites après l'administration du composé par rapport à la surface de la papule de référence, et est exprimée en pour-cent.

Dans le tableau V ci-dessous, on donne l'activité antihistaminique obtenue pour le composé P. Dans ce tableau,

la première colonne indique le temps, exprimé en heures, écoulé depuis l'administration du composé testé;
la deuxième colonne, la surface, exprimée en $mm^2$, des papules induites par l'histamine (moyenne observée sur les 9 chiens ± déviation standard);
la troisième colonne, la diminution (en pour-cent) de la surface des papules observée au cours du temps, par rapport à la surface de référence et
la quatrième colonne, la signification statistique de l'effet observé au cours du temps évaluée au moyen du test de Wilcoxon.

TABLEAU V

| Temps (heures) | Surface des papules ($mm^2$) | Diminution de la surface (%) | Valeur statistique. |
|---|---|---|---|
| 0 | 76 ± 8 | 100 | |
| 0,5 | 65 ± 10 | 85 | p≤0,01 |
| 1,5 | 44 ± 12 | 58 | p≤0,001 |
| 3 | 33 ± 10 | 43 | p≤0,001 |
| 6 | 41 ± 13 | 54 | p≤0,001 |
| 9 | 41 ± 10 | 54 | p≤0,001 |
| 12 | 41 ± 10 | 54 | p≤0,001 |
| 24 | 45 ± 5 | 59 | p≤0,001 |
| 32 | 51 ± 5 | 67 | p≤0,01 |

On constate que la diminution de la surface des papules observée 30 minutes après l'administration du composé P est de 15 %. L'inhibition maximale est observée après 3 heures et atteint 57 %.
Après 32 heures, on observe encore une inhibition statistiquement significative de 33 %.

4. Toxicité

Les composés de formule V présentent une faible toxicité. La dose létale (induisant la mort de 2 souris sur 3 lors d'une injection intrapéritonéale des composés) est nettement supérieure à la dose qui inhibe la réaction cutanée induite par l'histamine chez le chien. Dans le tableau VI, on donne les valeurs des doses létales pour quelques composés de formule V.

TABLEAU VI

| Composé | Dose létale (mole/kg) |
|---|---|
| C | > $1.10^{-3}$ |
| D | > $1.10^{-3}$ |
| E | $1.10^{-3}$ |
| F | > $1.10^{-3}$ |
| G | $6.10^{-4}$ |
| H | $6.10^{-4}$ |
| I | $1.10^{-4}$ |
| J | $1.10^{-4}$ |
| K | $3.10^{-4}$ |
| L | $1.10^{-3}$ |
| O | $3.10^{-4}$ |
| P | $3.10^{-4}$ |

5. Posologie et administration.

Les composés de formule V ont en particulier des activités antiallergique, antihistaminique d'une part, tranquillisante et anxiolytique d'autre part. Les compositions pharmaceutiques renfermant ces composés peuvent être administrées oralement, par voie parentérale ou rectale; elles peuvent aussi être administrées en spray ou instillations

nasales (aérosols) ou sous forme de crèmes ou d'onguent.

Pour l'administration orale, on utilisera des formes solides ou liquides telles que comprimés, gelules, dragées, granulés, solutions, sirops etc...

Pour l'administration parentérale, on utilisera par exemple des solutions, suspensions ou émulsions aqueuses ou huileuses.

Pour l'administration rectale, on fait usage de suppositoires.

Les formes pharmaceutiques citées ci-dessus sont préparées par des méthodes couramment utilisées par les pharmaciens et peuvent contenir les adjuvants traditionnels en doses pharmaceutiquement non toxiques, tels que dispersants, stabilisants, agents conservateurs, édulcorants, colorants, etc...

Le pourcentage de matière active peut varier selon les formes pharmaceutiques dans de larges limites, dépendantes du mode d'administration et en particulier de la fréquence d'administration. Quant à la posologie journalière, elle peut varier dans une large gamme s'étendant de 0,5 à 100 mg de matière active, de préférence entre 2 et 20 mg.

## Revendications

1. Enantiomères lévogyre et dextrogyre de 1-[(4-chlorophényl)phénylméthyl]-4-[(4-méthylphényl)sulfonyl]pipérazine de formule

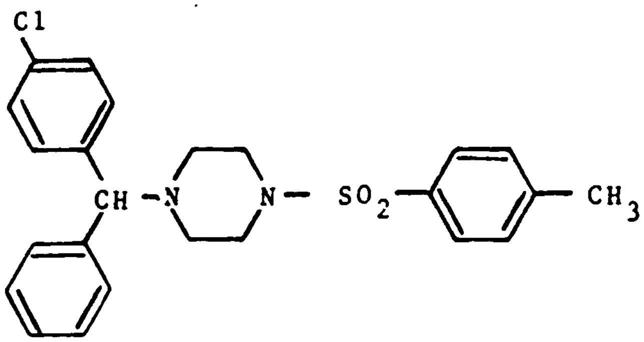

(I)

2. Procédé de préparation des énantiomères lévogyre et dextrogyre de la 1-[(4-chlorophényl)phénylméthyl]-4-[(4-méthylphényl)sulfonyl]pipérazine de formule I selon la revendication 1, caractérisé en ce que l'on fait réagir un énantiomère de la (4-chlorophényl)phénylméthylamine de formule

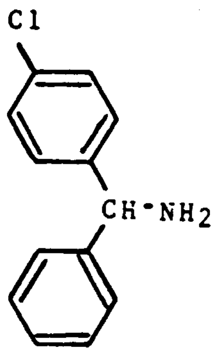

(II)

avec un N,N-diéthyl-4-méthylbenzènesulfonamide de formule

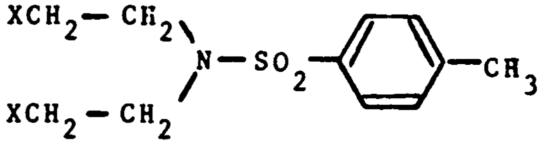

(III)

dans laquelle X représente un atome de chlore, de brome ou d'iode, ou le groupe (4-méthylphényl)sulfonyloxy ou méthylsulfonyloxy, en présence de 2,2 à 4,4 équivalents d'une base organique ou minérale par équivalent de l'énantiomère de la (4-chlorophényl)phénylméthylamine et à la température d'ébullition du mélange réactionnel.

3. Procédé selon la revendication 2, caractérisé en ce que la base est choisie dans le groupe consistant en l'éthyldiisopropylamine, la N-éthylmorpholine, la 2,4,6-triméthylpyridine, la triéthylamine et un carbonate de métal alcalin.

4. Procédé selon l'une quelconque des revendications 2 et 3, caractérisé en ce que la base est l'éthyldiisopropylamine.

5. Utilisation des énantiomères lévogyre et dextrogyre de la 1-[(4-chlorophényl)phénylméthyl]-4-[(4-méthylphényl)sulfonyl]pipérazine de formule I, selon la revendication 1, pour la préparation des énantiomères de la 1-[(4-chlorophényl)phénylméthyl]pipérazine de formule

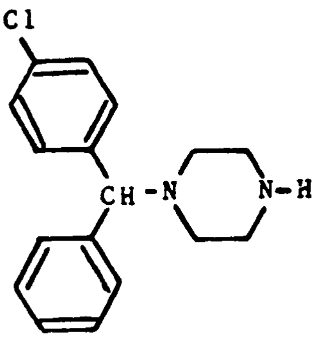

(IV)

par hydrolyse par l'acide bromhydrique, en milieu acide acétique, en présence d'un composé phénolique, de préférence l'acide 4-hydroxybenzoïque.

6. Procédé de préparation des énantiomères lévogyre et dextrogyre de la 1-[(4-chlorophényl)phénylméthyl]pipérazine de formule

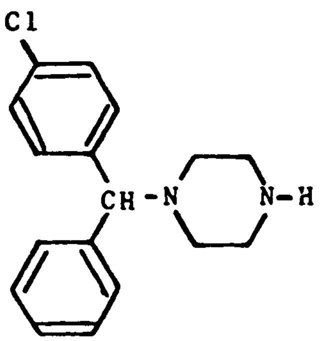

(IV)

caractérisé en ce que l'on hydrolyse un énantiomère de la 1-[(4-chlorophényl)phénylméthyl]-4-[(4-méthylphényl)sulfonyl]pipérazine de formule

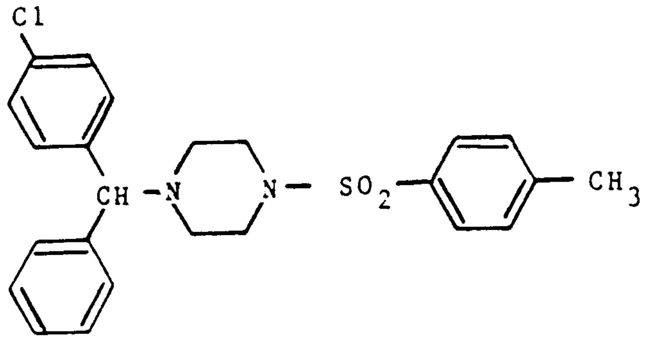

(I)

par l'acide bromhydrique, en milieu acide acétique, en présence d'un composé phénolique, de préférence l'acide 4-hydroxybenzoïque et à une température comprise entre 18 et 100°C.

**Claims**

1. Laevorotatory and dextrorotatory enantiomers of 1-[(4-chlorophenyl)phenylmethyl]-4-[(4-methylphenyl)sulfonyl]piperazine of formula

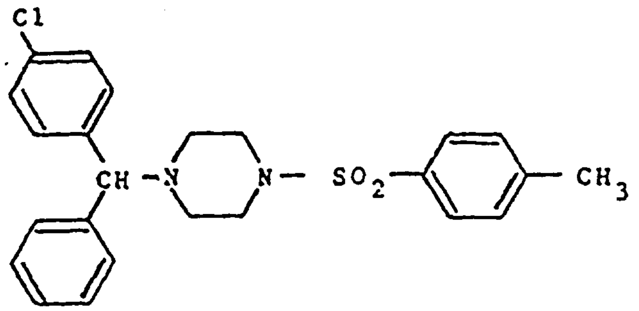

(I)

2. Method for preparing the laevorotatory and dextrorotatory enantiomers of 1-[(4-chlorophenyl)phenylmethyl]-4-[(4-methylphenyl)sulfonyl]piperazine of formula I according to claim 1, characterized in that an enantiomer of (4-chlorophenyl)phenylmethylamine of formula

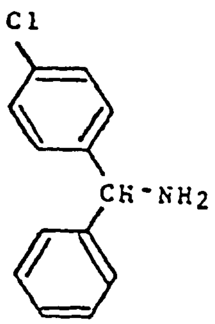

(II)

is reacted with an N,N-diethyl-4-methylbenzenesulfonamide of formula

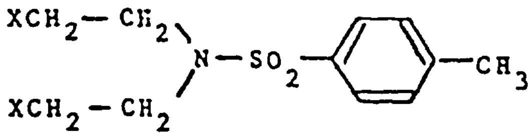

(III)

in which X represents a chlorine, bromine or iodine atom, or the (4-methylphenyl)sulfonyloxy group or the methylsulfonyloxy group, in the presence of 2.2 to 4.4 equivalents of an organic or inorganic base per equivalent of the enantiomer of (4-chlorophenyl)phenylmethylamine and at the boiling point of the reaction mixture.

3. Method according to claim 2, characterized in that the base is chosen from the group consisting of ethyldiisopropylamine, N-ethylmorpholine, 2,4,6-trimethylpyridine, triethylamine and an alkali metal carbonate.

4. Method according to either of claims 2 or 3, characterized in that the base is ethyldiisopropylamine.

5. Use of the laevorotatory and dextrorotatory enantiomers of 1-[(4-chlorophenyl)phenylmethyl]-4-[(4-methylphenyl)sulfonyl]piperazine of formula I according to claim 1, for the preparation of the enantiomers of 1-[(4-chlorophenyl)phenylmethyl]piperazine of formula

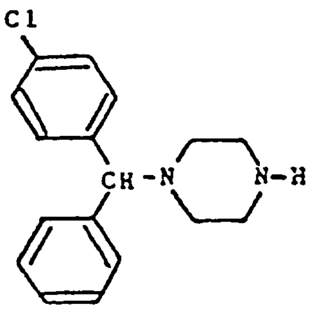

(IV)

by hydrolysis with hydrobromic acid, in an acetic acid medium, in the presence of a phenolic compound, preferably 4-hydroxybenzoic acid.

6. Method for preparing the laevorotatory and dextrorotatory enantiomers of 1-[(4-chlorophenyl)phenylmethyl]piperazine of formula

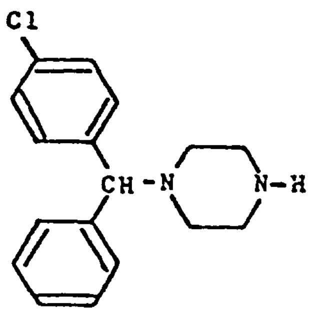

(IV)

characterized in that an enantiomer of 1-[(4-chlorophenyl)phenylmethyl]-4-[(4-methylphenyl)sulfonyl]piperazine of formula

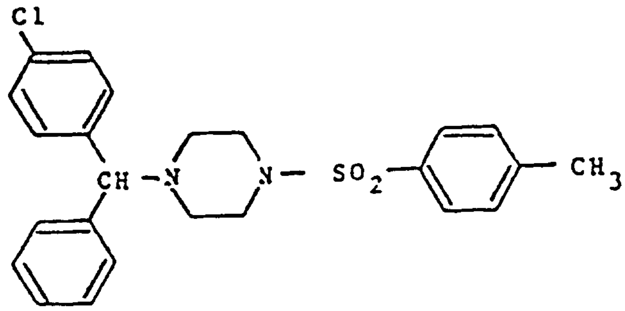

(I)

is hydrolysed with hydrobromic acid, in an acetic acid medium, in the presence of a phenolic compound, preferably 4-hydroxybenzoic acid, at a temperature comprised between 18 and 100°C.

**Patentansprüche**

1. Links- und rechtsdrehendes Enantiomer von 1-[(4-Chlorphenyl)phenylmethyl]-4-[(4-methylphenyl)sulfonyl]piperazin der Formel

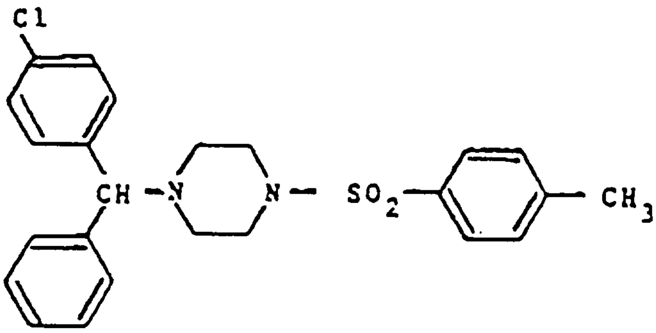

(I)

2. Verfahren zur Herstellung des links- und rechtsdrehenden Enantiomers des 1-[(4-Chlorphenyl)phenylmethyl]-4-[(4-methylphenyl)sulfonyl]piperazins der Formel I gemäß Anspruch 1, dadurch gekennzeichnet, dass man ein Enantiomer des (4-Chlorphenyl)phenylmethylamins der Formel

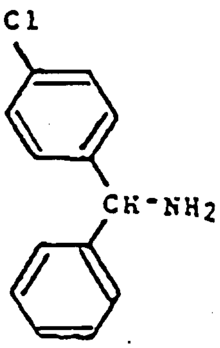

(II)

mit einem N,N-Diethyl-4-methylbenzolsulfonamid der Formel

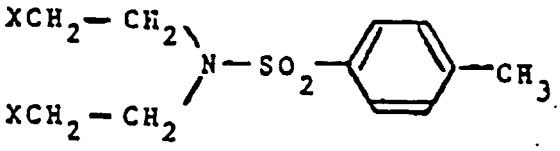

(III)

in der X ein Chlor-, Brom- oder Iodatom oder die (4-Methylphenyl)sulfonyloxy- oder Methylsulfonyloxygruppe darstellt, in Gegenwart von 2,2 bis 4,4 Equivalenten einer organischen oder mineralischen Base pro Equivalent des Enantiomers des (4-Chlorphenyl)phenylmethylamins und bei der Siedetemperatur des Reaktionsgemischs umsetzt.

3. Verfahren gemäß Anspruch 2, dadurch gekennzeichnet, dass die Base aus der Gruppe ausgewählt ist, die aus Ethyldiisopropylamin, N-Ethylmorpholin, 2,4,6-Trimethylpyridin, Triethylamin und einem Alkalimetallcarbonat besteht.

4. Verfahren gemäß einem der Ansprüche 2 und 3, dadurch gekennzeichnet, dass die Base Ethyldiisopropylamin ist.

5. Verwendung des links- und rechtsdrehenden Enantiomers des 1-[(4-Chlorphenyl)phenylmethyl]-4-[(4-methylphenyl)sulfonyl]piperazins der Formel I, gemäß Anspruch 1 zur Herstellung der Enantiomere des 1-[(4-Chlorphenyl)phenylmethyl]-piperazins der Formel

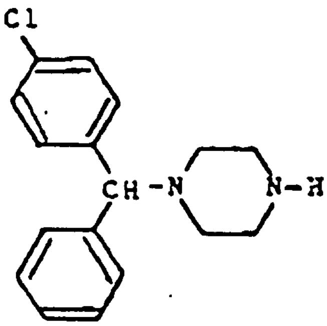

(IV)

durch Hydrolyse durch Bromwasserstoffsäure in essigsaurem Medium in Gegenwart einer phenolischen Verbindung, vorzugsweise 4-Hydroxybenzoesäure.

6. Verfahren zur Herstellung des links- und rechtsdrehenden Enantiomers des 1-[(4-Chlorphenyl)phenylmethyl]-piperazins der Formel

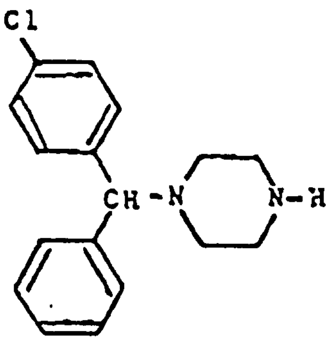

(IV)

dadurch gekennzeichnet, dass man ein Enantiomer des 1-[(4-Chlorphenyl)phenylmethyl]-4-[(4-methylphenyl)sulfonyl]piperazins der Formel

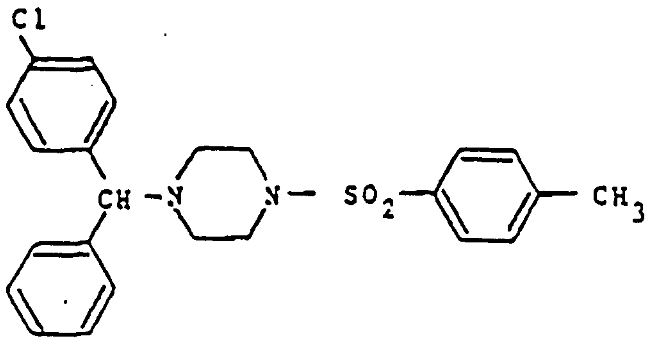

(I)

durch Bromwasserstoffsäure in essigsaurem Medium in Gegenwart einer phenolischen Verbindung, vorzugsweise 4-Hydroxybenzoesäure, und bei einer Temperatur zwischen 18 und 100 °C hydrolysiert.